# EUROPEAN PATENT APPLICATION

(11) **EP 3 812 466 A1**
(43) Date of publication of application: **28.04.2021**
(21) Application number: 19205177.9
(22) Date of filing: 24.10.2019
(51) Int. Cl.: C12P 7/10, C12P 19/14

(54) **A METHOD FOR FORMING A STORAGE STABLE HYDROLYSATE FROM A LIGNOCELLULOSIC MATERIAL**

(71) Applicant: Sekab E-Technology AB, 891 26 Örnsköldsvik (SE)
(72) Inventor: CAVKA, Adnan, 891 96 ARNÄSVALL (SE); SUNDVALL, Elias, 892 32 DOMSJÖ (SE)
(74) Representative: Kransell & Wennborg KB

(57) **Abstract**

The present disclosure generally relates to a method and a system for forming a storage stable hydrolysate from a lignocellulosic material and to a hydrolysate formed by such a method. It also relates to the use of the hydrolysate to reduce and/or control microbial contamination during storage and/or fermentation. Additionally, the present disclosure relates to a method and a system for reducing and/or controlling microbial contamination in a separate hydrolysis and fermentation (SHF) process.

## Description

### TECHNICAL FIELD

The present disclosure generally relates to a method and a system for forming a storage stable hydrolysate from a lignocellulosic material and to a hydrolysate formed by such a method. It also relates to the use of the hydrolysate to reduce and/or control microbial contamination during storage and/or fermentation. Additionally, the present disclosure relates to a method and a system for reducing and/or controlling microbial contamination in a separate hydrolysis and fermentation (SHF) process.

### BACKGROUND

Biorefineries producing commodities from renewable resources offer an alternative to oil refineries based on dwindling supplies of petroleum and permit a move towards improved energy security. Lignocellulosic residues from forestry and agriculture are attractive as feedstocks, since they are abundant, relatively inexpensive, and are not used for food. Lignocellulose consists mainly of lignin and two classes of polysaccharides, cellulose and hemicellulose. The polysaccharides can be hydrolyzed to sugars and converted to various fermentation products, such as bioalcohols, in processes based on biocatalysts, such as the industrially important baker's yeast *Saccharomyces cerevisiae.*

The hydrolysis of cellulose is typically preceded by a pretreatment, in which the hemicellulose is degraded and the cellulose is made increasingly accessible to cellulolytic enzymes. During hydrolysis, the cellulose present is partly converted into reducing sugars. The hydrolysis is a relatively long process, and may last up to 150 hours.

In the subsequent fermentation step, remaining cellulosic material is converted into reducing sugars. The fermentation is finished when the cellulosic material has been converted into fermentable sugars, and such fermentable sugars may be converted into target biochemicals, such as bioalcohols or acids and carbon dioxide. The fermentation step may take up to several days before being completed.

Microbial contamination is a widespread problem both during hydrolysis and during fermentation. Various species of *Lactobacillus sp.* are generally the predominant bacterial contaminants. Lactobacillus bacteria are known to be well adjusted to survival under conditions that most other bacteria are not, such as high ethanol concentrations, low pH and low oxygen conditions, commonly employed during fermentations. Undesirable presence of this type of contaminants is commonly controlled with the use antibiotics, which can cause not only waste disposal issues but also risk of introducing antibiotics resistance. Other types of organisms are also known to infect fermentations, such as wild yeast and fungi, but these are less prevalent than *Lactobacillus sp.*

A process wherein hydrolysis and fermentation are performed in separate steps, potentially under different process conditions, such as at a different pH and temperature, may be referred to as a separate hydrolysis and fermentation (SHF) process.

In an SHF process, the process may be discontinued between the hydrolysis and the fermentation steps. Under certain circumstances, it may be desirable to store the hydrolysate during a period of time prior to fermentation. The hydrolysate may for example need to be transported to a different location for subsequent fermentation.

During storage of the hydrolysate, the risk of bacterial growth is increased, which may result in an infected sugar hydrolysate. The introduction of such infected hydrolysate into the fermentation vessel may significantly impair the efficiency of the fermentation. The yield of the target chemical may be reduced as the *Lactobacilli* can inhibit fermenting microorganisms such as *Saccharomyces* sp., *Escherichia coli,* or desirable strains of *Lactobacillus sp.* An infected hydrolysate may also result in that the fermentation process must be shut down or delayed, causing loss of revenue and increased production costs.

There is therefore a need for improved methods to overcome problems with infections during hydrolysis and/or during subsequent fermentation. Particularly, there is a need to provide a hydrolysate from a lignocellulosic material, which is free from bacterial contaminants and which is suitable for storage and transport prior to fermentation.

### SUMMARY

In view of the above mentioned problems, it is an object of the present disclosure to provide improvements with respect to reducing and/or controlling contamination in a hydrolysate formed from a lignocellulosic material, and in a separate hydrolysis and fermentation (SHF) process. Particularly, the hydrolysate should be suitable for storage and transportation before fermentation.

According to a first aspect of the present disclosure, there is provided a method for forming a storage stable hydrolysate from a lignocellulosic material comprising:
a) pretreating the lignocellulosic material to form a pretreated lignocellulosic composition comprising a liquid component and a solid component; the solid component comprising at least lignin and cellulose
b) removing at least 80%, preferably at least 90% of the liquid component from the pretreated lignocellulosic composition to form a separated lignocellulosic component,
c) diluting the lignocellulosic component with a dilution liquid to form an aqueous slurry
d) subjecting the aqueous slurry to hydrolysis in the presence of at least one saccharification enzyme to form a hydrolysate,
e) adding an antimicrobial compound to the hydrolysate in an amount sufficient to form a storage stable hydrolysate, wherein the antimicrobial compound comprises at least one sulfur oxyanion.

As used herein, the term "storage stable hydrolysate" means a hydrolysate that may be stored for at least three weeks, preferably at least five weeks, such as at least eight weeks at room temperature without getting infected. In other words, the storage stable hydrolysate is substantially free from bacterial contaminants, and may therefore be safely introduced in a subsequent fermentation unit. The hydrolysate of the present invention provides an unfavorable environment for bacterial growth and survival, and is capable of maintaining its storage stable properties even during long periods of storage and transportation. If the hydrolysate is stored at 4°C, the storage period may be significantly longer.

A "lignocellulosic material" refers to a material comprising solely cellulose, combination of cellulose, lignin and possibly hemicellulose. The lignocellulosic material may for example be wood residues or forestry residues, such as wood chips, sawmill or paper mill discards, or agricultural residues, such as sugarcane bagass.

"Pretreating the lignocellulosic material" refers to pretreating a lignocellulosic material in order to modify its properties such that the cellulose becomes more accessible during subsequent hydrolysis. The pretreatment may involve one or several pretreatment methods known to the skilled person. The pretreatment can be made by hydrothermal/chemical, physical and biological methods. For example, the pretreatment may involve acid pretreatment, alkali pretreatment, ammonia fiber explosion, acid catalyzed nor non catalyzed steam explosion, autohydrolysis, organosolvents, sulfite or kraft pulping. Further, the pretreatment may include impregnation, which refers to absorption or mass transfer of an impregnation fluid or gas to the lignocellulosic material, followed by above exemplified processing and/or pre-treatment. The fluid may e.g. be solvents or solutions of acids such as sulphurous acid, sulphuric acid, ammonia, sulfite/bisulfite, sulfate hydroxides, and alcohols. The impregnation may also be performed using gas, such as a SO₂-gas or CO₂-gas, or with the combination of a gas and fluid of any given combination, concentration and proportion. The pretreatment may also comprise steaming. Steaming refers to a process used to drive air out from the cellulosic biomass to facilitate further hydrolysis of the cellulose. Steaming is a well-known method for pretreating e.g. lignocellulosic biomass.

The "solid component" of the pretreated lignocellulosic composition may comprise solid, unhydrolyzed or undissolved materials of the lignocellulosic material, such as lignin, cellulose, and/or hemicellulose.

The "liquid component" of the pretreated lignocellulosic composition may comprise water, one or more monosaccharides, oligosaccharides, and by-products of the pre-treatment process, such as hydrolysis and fermentation inhibitors, impregnation components and organic acids. Examples of hydrolysis and fermentation inhibitors include phenolic compounds, aliphatic acids, and furan aldehydes. These compounds may inhibit enzymes and yeast, during hydrolysis and fermentation, respectively.

The components comprised in the liquid component, particularly hydrolysis and fermentation inhibitors may have a negative effect on the efficiency of the hydrolysis and the fermentation process. The liquid component comprising such compounds is therefore removed from the pretreated lignocellulosic composition, as defined in step b) above. The removed liquid component may e.g. be utilized in or outside the process of the present disclosure, for example in ethanol fermentation (which typically does not require any separation or washing between the pretreatment and hydrolysis steps).

The step of removing the liquid component may include washing and/or centrifugation of the pretreated lignocellulosic composition. In embodiments, the method according to the present disclosure comprises an additional washing step after the liquid component has been removed from the pretreated lignocellulosic composition in step b.

The "separated lignocellulosic component" comprises the solid component of the pretreated lignocellulosic composition (i.e. unhydrolyzed or undissolved lignin, cellulose and/or hemicellulose), and small amounts of the liquid component. The lignocellulosic component comprises a ratio of solid component-to-liquid component of 80:20, e.g. 90:10, e.g. 95:5. The lignocellulosic component is substantially solid.

The separated lignocellulosic component is subsequently diluted with a dilution liquid to form an aqueous slurry. The dilution liquid is typically water or a sufficiently clean process liquid. For example, process liquid (e.g. washing liquor) recirculated from an upstream or downstream point of the process, may be reused and utilized. Preferably, the dilution liquid is water.

The aqueous slurry formed in step c) is substantially free from hydrolysis and fermentation inhibitors, and other detrimental compounds.

The aqueous slurry may thus be regarded as a "clean" or purified stream, wherein an appropriate amount of harmful by-products and hydrolysis and fermentation inhibitors have been removed. The aqueous slurry may comprise minor amounts of the liquid component of the pretreated cellulose composition formed in step a) of the method.

The aqueous slurry comprises less than 20% by weight, preferably less than 10% by weight and more preferably less than 5% by weight of the liquid component of the pretreated lignocellulosic composition.

In this regard, it should be noted that the addition of antimicrobial compounds comprising sulfur oxyanions to pre-treated lignocellulosic material that has not been subject to separation would have a detoxifying effect; i.e. reducing the amount of fermentation inhibitors, such as phenolic compounds, aliphatic acids, and furan aldehydes and generally improving the conditions during a subsequent fermentation process. However, the addition of sulfur oxyanions to a non-separated pretreatment stream could in fact increase the growth of bacteria, as illustrated by Example 1 below. In order to yield an antimicrobial effect for such non-separated streams, a significant amount of sulfur oxyanions would be required. The introduction of too high amounts of sulfur oxyanions in this step is associated with unnecessary costs and material expendings.

Accordingly, the separation, optional wash and subsequent dilution allows for the formation of a sugar rich aqueous slurry wherein less inhibitory compounds are present.

The aqueous slurry of step c) is then subjected to hydrolysis (step d) in the presence of at least one saccharification enzyme to form a hydrolysate.

The hydrolysis reaction is catalyzed by at least one saccharification enzyme. A "saccharification enzyme" refers to at least one enzyme that can convert or hydrolyze (ligno) cellulosic material into fermentable saccharides, such as monosaccharides and/or disaccharides. Such saccharification enzymes may be glycosidases, which hydrolyze polysaccharides. Examples of glycosidases include cellulose-hydrolyzing glycosidases, such as cellulases, endoglucanases, exoglucanases, cellobiohydrolases and β-glucosidases, hemicellulose hydrolysing glycosidases, such as xylanases, endoxylanases, exoxylanases, β-xylosidases, arabinoxylanases, mannanases, galactanases, pectinases and glucuronases, and starch hydrolysing glycosidases, such as amylases, cc- amylases, β-amylases, glucoamylases, cc-glucosidases and isoamylases, or any enzymes in the group of enzymes found in EC 3.2.1.x, such as EC 3.2.1.4, where EC is the Enzyme Commission number.

The hydrolysate formed in the process of the present disclosure may comprise sugars from lignocellulosic biomass, such as glucose, mannose, xylose, arabinose, galactose, sucrose and fructose.

In embodiments, the storage stable hydrolysate comprises at least glucose.

In embodiments, the storage stable hydrolysate comprises at least two different sugars.

The hydrolysis may be carried out at a pH in the range of 3 to 6, e.g. between 5 and 5.5. The temperature during hydrolysis may be in the range of from 40 to 70°C, e.g. between 50 and 60°C. These conditions allow the saccharification enzyme(s) to perform optimally. Furthermore, these conditions prevent the growth of undesirable microorganisms.

The method further comprises a step e) of adding an antimicrobial compound to the hydrolysate in an amount sufficient to form a storage stable hydrolysate, wherein the antimicrobial compound comprises at least one sulfur oxyanion.

Examples of antimicrobial compounds comprising at least one sulfur oxyanions are disulfite, bisulfite, dithionite, dithionate, sulfate, sulfite and thiosulfate.

The inventors have found that a method according to the present disclosure yields a stable and substantially "infection free" hydrolysate, wherein the presence of bacterial contaminants is eliminated, or substantially reduced. Such hydrolysate therefore allows for prolonged storage periods. A storage stable hydrolysate of the present disclosure may be stored up to three weeks, e.g. at least five weeks, such as at least eight weeks at room temperature without getting infected. If the hydrolysate is stored at 4°C, the storage period may be significantly longer. The hydrolysate may be used in a subsequent fermentation step, to provide a safe and contamination free fermentation. Furthermore, only small amounts of antimicrobial compound need to be added to yield a significant contamination reducing effect. The fact that the method involves a separation step prior to hydrolysis, and that only relatively pure sugar streams are utilized, allows the addition of antimicrobial compounds to "boost" the hydrolysate and preserve its infection free properties such that the hydrolysate becomes resistant to infections during storage and during subsequent fermentation.

In embodiments, the aqueous slurry formed in step c) has a suspended solids content of from 10 % to 35 % by weight. Depending on the fermentation process and what conditions and target chemicals that are to be produced, the dilution and suspended solids content may be adjusted. If the suspended solid content is too high, a larger amount of enzymes would be required in the subsequent hydrolysis step. This would significantly increase production costs.

A "target chemical" may be any chemical that can be prepared from pretreated and hydrolyzed cellulosic material in a process comprising fermentation. The target chemical may e.g. be alcohols or acids, alkanes, alkenes, aromatics, aldehydes, ketones, biopolymers, proteins, peptides, amino acids, vitamins, antibiotics and other pharmaceuticals. For example, the target chemical is lactic acid, butanol, succinic acid or isobutene. Ethanol is typically not the target chemical in the method of the present disclosure, since the production of ethanol does not require any separation between the pretreatment and hydrolysis steps.

In embodiments, the hydrolysis step b) is performed in the presence of oxygen. Preferably, the hydrolysis step is performed in the presence of air.

In other words, the hydrolysis is an aerobic process. This is beneficial as the hydrolysis of cellulose is improved. Enzymatic oxidation of cellulose reduces the forces holding the cellulose fibers together, which thereby increases the availability and accessibility for the hydrolytic enzymes to hydrolyze cellulose to sugars.

It is conceivable to add the antimicrobial compound prior to or during hydrolysis, however if the hydrolysis is performed in the presence of air (and temperatures of 40°C or more), the sulfite oxyanions will disassociate and form gaseous sulfur dioxide (SO₂) which will leave the hydrolysis with the air flow. In other words, the antimicrobial effect would diminish. Therefore, the addition of sulfur oxyanions is preferably made after hydrolysis.

In embodiments, the concentration of the antimicrobial compound is between 1 and 100 mM, e.g. between 5 and 50 mM, e.g. between 5 and 25 mM. Preferably, the concentration is in the lower range as a too high concentration may inhibit microbes utilized in a subsequent fermentation step.

As demonstrated by example 2 hereinafter, only small amounts of antimicrobial compounds are required to achieve an antimicrobial effect. This is mainly because the hydro lysate formed by the process of the present disclosure is substantially pure, and contains less inhibitors, and other compounds that may react and thus consume the oxyanions, which is the desired effect when such sulfur oxyanion compounds are used for conditioning or detoxification of lignocellulosic hydrolysates containing hydrolysis and fermentation inhibitors.

In embodiments, the antimicrobial compound is selected from dithionite and sulfite.

Dithionite and sulfite are known to be used as reducing agents to improve the fermentability during fermentation, typically by means of alleviating the effect of fermentation inhibitors. This is for instance disclosed in WO2011/080129.

Sulfite (SO₃²⁻) is used in several large-scale industrial processes. Dithionite ([S₂O₄]²⁻) is an industrial chemical used in the pulp and paper industry for reductive bleaching and in the textile industry as a reducing agent in dyeing processes. Hence, both sulfite and dithionite may be available in large quantities. Further, it is to be understood the antimicrobial agent may comprise sulfite and/or dithionite in salt form, i.e. complexed with different cations. Examples include Na₂SO₃, NaHSO₃, KHSO₃, and Na₂S₂O₄.

Dithionite may be added in an amount of 1-50, e.g. from 4 to 25 mM.

Sulfite may be added in an amount of 1-100, e.g. from 10 to 60 mM.

In embodiments, the method of the present disclosure further comprises the step of subjecting the hydrolysate formed in step d) or step e) to separation to remove at least a portion of residual solid components formed during the hydrolysis.

Such solid components may e.g. include incomplete hydrolyzed cellulose and/or lignin. Preferably, the hydrolysate comprises less than 10% of such solid components.

In embodiments, the method further comprises the step of controlling the amount of antimicrobial compound in the storage stable hydrolysate by:
- measuring the sulfur ion content in the storage stable hydrolysate
- comparing the sulfur ion content with a reference sulfur value, and optionally
- adding an additional amount of the antimicrobial compound if the sulfur ion content is lower than the reference sulfur value.

In embodiments, the sulfur ion content is measured inline; i.e. the measurements are performed directly in the process line.

In alternative embodiments, the sulfur ion content is measured online; i.e. the measurements are performed on a sample diverted from the process line, such as in a bypass loop from the main process line.

Inline and online sulfur ion measurements are advantageous since time consuming manual sampling methods and subsequent laboratory analyses used for controlling process parameters can be avoided. In other words, inline and online sulfur ion measurements provide a swift and efficient approach for correcting an ongoing process.

The sulfur ion content may be measured by titration means, a sulfur probe or a sulfur sensor, which is configured to reflect the content of sulfur ions; i.e. sulfur oxyanions present in the hydrolysate.

The measured sulfur content is compared with a reference sulfur value, which may vary depending on the process conditions or the target chemical to be produced during subsequent fermentation. The reference sulfur value may be a predetermined threshold value.

If the sulfur ion content is lower than the reference sulfur value, additional sulfur oxyanions, e.g. more dithionite and/or sulfite may be added. For example, if less than 1 mM sulfur is present in the sample, more sulfite/dithionite may need to be added.

The measurement may be made directly after the introduction of the antimicrobial compound to ensure the quality and an efficient infection control of the sample.

The measurement(s) may also be performed during storage of the formed hydrolysate or directly before fermentation to make sure that the hydrolysate to be added to the fermentation vessel is free from contaminants.

According to a second aspect, there is provided a hydrolysate formed by the method as described hereinbefore.

After the hydrolysate has been formed, it may be stored before being fermented and processed to a target chemical. The hydrolysate of the present disclosure is adapted to remain stable and free from bacterial contaminants, and is therefore suitable for storage during relatively long periods of time, and for being transported to e.g. a different fermentation site.

According to third aspect, there is provided the use of a hydrolysate formed by the method as described hereinbefore to reduce and/or control microbial contamination during storage and/or fermentation to a target chemical.

As used herein, "reduce and/or control microbial contamination" means that undesired microbial contaminants are kept at a minimum or a reduced level throughout the storage and fermentation periods. Bacterial infections commonly occurring during storage and fermentation are thus prevented.

According to a fourth aspect, there is provided a method for reducing and/or controlling microbial contamination in a separate hydrolysis and fermentation (SHF) process comprising:
- providing a storage stable hydrolysate according to the second aspect above or formed by the method of the first aspect above,
- subjecting the storage stable hydrolysate to fermentation.

Fermentation of the hydrolysate may be performed by a fermenting organism; i.e. an organism that is capable of fermenting saccharides into a target chemical. The fermenting organism may be at least one eukaryotic or prokaryotic microorganism, such as bacteria and/or yeast. Examples of bacteria and yeasts which are capable of fermenting saccharides into other chemical compounds are known to the skilled person. Bacteria from *Escherichia, Lactobacillus* and *Streptomyces* may be used for the fermentation. Yeasts from *Saccharomyces, Pichia* and *Candida* may be used as the fermenting organism. The fermenting organism may for example be wild type, mutant or recombinant *Saccharomyces cerevisiae.* Using *S. cerevisiae* for producing a target chemical by means of fermentation is advantageous since *S. cerevisiae* is well established with regard to industrial fermentation and provides for a high product yield.

Preferably, the fermentation is a fed-batch or a continuous fermentation.

In other words, the fermentation is semi-continuous or continuous. Since the hydrolysate stream is added in a (semi) continuous manner during fermentation, it is highly important to secure the infection control in the hydrolysate stream. The continuous introduction of an infected hydrolysate may significantly impair the fermentation.

For example, a fed-batch fermentation process comprising at least one batch phase, feed phase and end phase, respectively, and having continuous addition of fermentation media during the at least one feed phase may be used.

In embodiments, the SHF process comprises steps for controlling the microbial fermentation in an automated or semi-automated fermenting process.

In other words, the fermentation process may comprise the steps of:
- continuous or semi-continuous addition, to a fermentation vessel, of the storage stable hydrolysate as described hereinbefore
- providing an initial active population of fermentation microorganisms into the fermentation vessel;
- online measuring of a residual sugar indicator parameter RSI, which parameter directly or indirectly indicates the concentration of residual sugars, during fermentation in the fermentation vessel;
- determining an RSI setpoint based on a rate of change of the measured residual sugar indicator parameter, such that the RSI setpoint corresponds to a maximum rate of change; and
- automatically adapting the amount of sugar added to the fermentation vessel in a predetermined manner in response to the measured residual sugar indicator parameter RSI and the RSI setpoint, so as to achieve and maintain the RSI setpoint, whereby efficient co-fermentation of sugars to fermentation products is obtained.

The RSI set point is a target parameter; i.e. a target value or an interval corresponding to desirable or optimum process conditions. The RSI setpoint may be fixed and/or variable. The determination of the RSI setpoint may involve calculations and/or online following of the registered RSI signal during the fermentation process. Preferably, the RSI setpoint corresponds to a steady state condition in the fermentation vessel.

In a controlled fermentation method as defined above, a favorable steady state condition is reached in the fermentation. The steady state condition is associated with in situ propagation of microorganisms, which renders additional inoculum unnecessary. The risk for infections is significantly reduced by utilizing a stable storage hydrolysate according to the present disclosure.

PCT/EP2019/060707 discloses a method and a system for controlling the fermentation process, the entire content which is incorporated herein by reference.

With the method of the present disclosure, it is possible to store the hydrolysate for a prolonged period of time.

In embodiments, the method further comprises the step of storing the hydrolysate at least three weeks, preferably at least five weeks, such as at least eight weeks at room temperature prior to fermentation.

This is advantageous, and allows for the SHF process to be discontinued after hydrolysis. The hydrolysate may thus be removed, stored and potentially transported to a different fermentation system site.

It should be noted that if the hydrolysate is stored at 4°C, the storage period may be significantly longer.

The method may further comprise the step of recovering or concentrating the hydrolysate prior to fermentation. Depending on the target chemical to be produced and the fermentation process conditions, the hydrolysate may be concentrated to increase the final sugar concentration of the hydrolysate.

The sugar concentration may be in the range of 8 to 75 %, e.g. from 20 to 70, preferably from 40 to 70 % depending on the application.

In other words, the sugar content of the hydrolysate may be from 80 g/l to 750 g/l, e.g. from 200 to 700 g/l, e.g. from 400 to 700 g/l.

According to fifth aspect, there is provided a system for forming a storage stable hydrolysate from a lignocellulosic material comprising:
- a pretreatment unit for pretreating the lignocellulosic material to form a pretreated lignocellulosic composition comprising a liquid component and a solid component; the solid component comprising at least lignin and cellulose
- means for removing at least 80%, preferably at least 90% of the liquid component from the pretreated lignocellulosic composition to form a separated lignocellulosic component,
- means for diluting the lignocellulosic component with a dilution liquid, e.g. water to form an aqueous slurry,
- a hydrolysis unit arranged to receive the aqueous slurry and to subject the aqueous slurry to hydrolysis in the presence of at least one saccharification enzyme to form a hydrolysate
- means for adding an antimicrobial compound to the hydrolysate to form a storage stable hydrolysate, wherein the antimicrobial compound comprises at least one sulfur oxyanion.

In embodiments, the system further comprises means for separating the hydrolysate to remove at least a portion of residual solid components formed during hydrolysis. Such separation means are preferably arranged in fluid communication with and downstream of the hydrolysis unit.

The hydrolysis unit may be configured to receive an air stream through an inlet of the hydrolysis unit, and to discharge the air stream by means of an outlet the hydrolysis unit.

Thereby, air is flushed through the hydrolysis unit and mixed with the aqueous slurry. This increases the efficiency of the enzymes in the hydrolysis unit, yielding a quicker and more efficient hydrolysis

In embodiments, the system of the present disclosure comprises means for controlling the amount of antimicrobial compound in the storage stable hydrolysate, comprising:
- means for measuring the sulfur ion content of the storage stable hydrolysate, and optionally
- means for adding additional antimicrobial compound in case the sulfur ion content is lower than a reference sulfur value.

The means for measuring the sulfur ion content may be titration, a sulfur probe or a sulfur sensor arranged to measure the sulfur content after the antimicrobial compound has been added to the hydrolysate.

According to a sixth aspect, there is provided a system for reducing and/or controlling microbial contamination in a separate hydrolysis and fermentation (SHF) process comprising:
- a system for forming a storage stable hydrolysate as described above
- at least one fermentation vessel arranged downstream of and in fluid connection with the system for forming a storage stable hydrolysate.

The fermentation vessel may comprise agitation means to mix the hydrolysate; i.e. the fermentation media with fermenting microorganisms.

Furthermore, the fermentation vessel may comprise means for measuring a residual sugar indicator parameter, RSI, which directly or indirectly indicates the concentration of residual sugars in the fermentation vessel. The measuring means may include one or more sensors arranged in the fermentation vessel or in a loop outside the vessel. Sensors may e.g. be arranged in the inlet and/or outlet of the fermentation vessel to provide RSI signals.

The RSI may be a density indicator, for example obtained using optical measurements or refractive index (RI) measurements. The RSI could also be derived from measurements and calculations of carbon dioxide (CO₂) generation, or be obtained by direct measurements of sugar concentration.

The fermentation vessel may further comprise means for determining and setting an RSI setpoint based on the rate of change of the measured residual sugar indicator parameter such that the RSI setpoint corresponds to a maximum rate of change, and control means for automatically adapting the amount of sugar added to the fermentation vessel in a predetermined manner in response to the measured residual sugar indicator parameter RSI and the RSI setpoint, so as to achieve and maintain the RSI setpoint, whereby efficient co-fermentation of sugars to fermentation products is obtained.

The amount of sugar added to the fermentation vessel may be automatically adapted in a predetermined manner in response to the measured residual sugar indicator parameter RSI and the RSI setpoint, so as to achieve and maintain the RSI setpoint, whereby efficient co-fermentation of sugars to fermentation products is obtained.

The system may further comprise means for controlling the amount of antimicrobial compound in the fermentation vessel, comprising:
- means for measuring the sulfur ion content in the fermentation vessel, and optionally
- means for adding additional antimicrobial compound in case the sulfur ion content is lower than a reference sulfur value.

Such means may be arranged within the fermentation vessel for securing infection control during fermentation.

In embodiments, the sulfur ion content is measured both prior to fermentation (i.e. to the storage stable hydrolysate) and during fermentation.

If the fermentation system also comprises means to control the microbial fermentation by means for measuring a residual sugar indicator parameter, RSI, the content of sulfur may be measured simultaneously with such RSI measurements.

If additional sulfur oxyanions are added in the fermentation vessel, this may also have a positive effect on the fermentation, e.g. it may increase the rate of the fermentation reaction or the total amount of target product produced in the fermentation reaction.

According to a sixth aspect, there is provided the use of dithionite or sulfite for decreasing microbial contamination of a hydrolysate formed from a lignocellulosic material.

### BRIEF DESCRIPTION OF THE DRAWINGS

The various aspects of the present disclosure, including its particular features and advantages, will be readily understood from the following detailed description and the accompanying drawings, in which:
Figure 1 schematically illustrates a separate hydrolysis and fermentation (SHF) process according to the present disclosure.
Figure 2 illustrates growth of *Lactobacillus* and production of lactic acid in a spruce hydrolysate detoxified with various concentrations of sodium sulfite.
Figure 3 illustrates the growth of *Lactobacillus* and production of lactic acid in a pure and sugar rich hydrolysate containing various concentrations of sodium sulfite.

### DETAILED DESCRIPTION

The present invention will now be described more fully hereinafter with reference to the accompanying drawings. The present invention may, however, be embodied in many different forms and should not be construed as limited to the embodiments set forth herein; rather, these embodiments are provided for thoroughness and completeness, and fully convey the scope of the present invention to the skilled person.

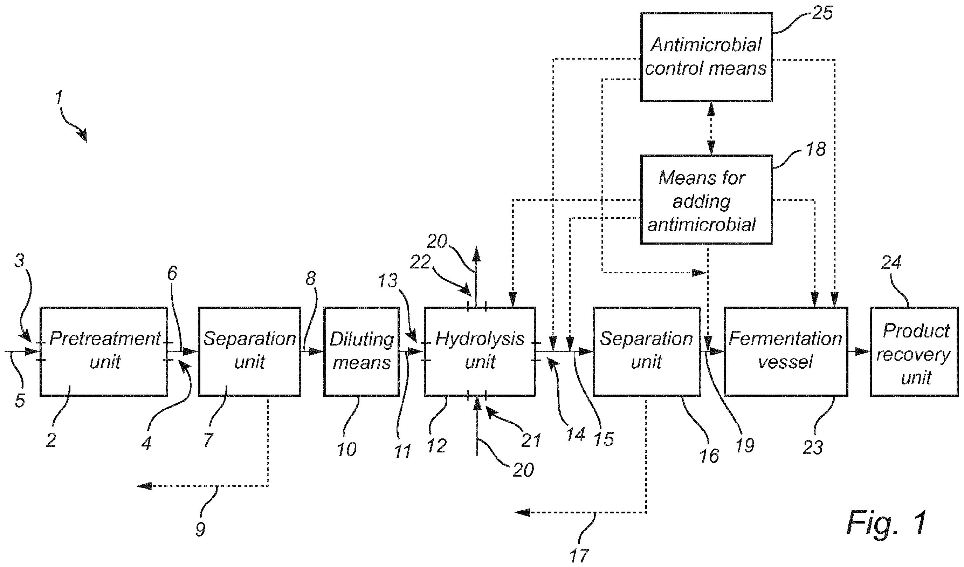

Figure 1 illustrates a system for reducing and/or controlling microbial contamination in a separate hydrolysis and fermentation (SHF) process. The system 1 comprises a pretreatment unit 2, e.g. comprising a reactor, a vessel or a container, wherein the lignocellulosic material is pretreated. The present disclosure is not limited to a particular type of pretreatment method, but any pretreatment method may be utilized. Typically, the temperature in the pretreatment unit 2 is from 150 to 230°C, the pH is from 1 to 3 and the pretreatment time may be from 3 minutes to 60 minutes. The pretreatment unit 2 comprises an inlet 3 and an outlet 4. A feed stream of lignocellulosic material 5 enters the pretreatment unit 2 through the inlet 3, and the pretreated lignocellulosic composition 6 is discharged through the outlet 4. The pretreated lignocellulosic composition 6 comprises a liquid component and a solid component (comprising lignin and cellulose and/or hemicellulose).

The system comprises means 7 to remove at least 80%, preferably at least 90% of the liquid component from the pretreated lignocellulosic composition 6 to form a separated lignocellulosic component 8. As illustrated in figure 1, this may be achieved by means of a separation unit 7 arranged downstream of and in fluid communication with the pretreatment unit 2. Alternatively, it may be arranged inside the pretreatment unit 2. The separation unit 7 is configured to separate the majority of the liquid component from the solid component. As the separation unit 7 removes the liquid component, it also functions as a dewatering unit. The separation unit 7 may comprise a screen, a filter, a decanter screw, a centrifuge or similar equpiment to separate and remove the liquid component from the pretreated lignocellulosic composition.

The liquid component is removed from the separation device, as illustrated by the liquid stream 9. The liquid component comprises water and byproducts formed during the pretreatment process. The liquid stream 9 may be utilized outside the process, e.g. for ethanol fermentation. The separated lignocellulosic component (comprising the solid component of the pretreated lignocellulosic composition) 8 is discharged from the separation unit 7 and is thereafter diluted.

Means 10 for diluting the lignocellulosic component 8 with a dilution liquid, preferably water, to form an aqueous slurry 11 may be performed in a separate dilution vessel or tank as illustrated in figure 1. Dilution can e.g. be accomplished by means of a dilution screw, or a mixer/pump/standpipe with dilution liquid addition. A stream of dilution liquid, e.g. water is added to the lignocellulosic component 8 downstream of the separation unit 7 such that an aqueous slurry 11 is formed. It is also conceivable that the stream of water is added to the separation unit 7 such that the separation unit 7 acts both to separate the "dirty" liquid component and to replace it with fresh water.

The system 1 further comprises a hydrolysis unit 12 for hydrolyzing the aqueous slurry, which has both been pretreated and cleaned; i.e. separated and diluted with water.

The hydrolysis unit 12 comprises an inlet 13 for receiving the aqueous slurry 11, and an outlet 14 for discharge of the hydrolysate 15 after the hydrolysis has been completed.

A second separation unit 16 may be arranged to receive the hydrolysate 15 in order to remove residual solids formed during hydrolysis. As illustrated in figure 1, residual solid components 17 are removed from the hydrolysate 15.

The system also comprises means 18 for adding antimicrobial compound to the hydrolysate 15 before separation in the separation unit 16 or to the separated hydrolysate stream 19. It is also conceivable to add the antimicrobial compound directly to the hydrolysis unit 12 upon completion of the hydrolysis reaction. Antimicrobial compound may, if needed, also be added to the fermentation vessel 23. The dotted arrows in figure 1 illustrates points in the process where the antimicrobial compound can be added.

The hydrolysis unit 12 may be configured to receive an air stream 20 through an inlet 21 and to discharge the air stream 20 through an outlet 22 of the hydrolysis unit 12.

In other words, the air stream 20 is mixed with the feedstock within the hydrolysis unit 12. This may be achieved by simultaneously stirring the feedstock in the hydrolysis unit 12. The air stream 20 allows for the enzymes present in the system to become more efficient, yielding a quicker and more efficient hydrolysis.

The air inlet 21 may be the same as the inlet 13 for receiving the aqueous slurry 11 and the air outlet 22 may be the same as the outlet 14 for discharging the hydrolysate stream 15. Alternatively, the air inlet 21 is arranged in a bottom portion of the hydrolysis unit 12 and the air outlet 22 is arranged in a top portion of the hydrolysis unit 12, as illustrated in figure 1.

The system 1 may be connected to a fermentation vessel 23 arranged in fluid communication with and downstream of the hydrolysis unit 12.

A product recovery unit 24, such as distillation or ion exchange chromatography may be connected to the system downstream of the fermentation vessel.

The system may also comprise means 25 for controlling the amount of antimicrobial compound. Such means 25 comprises means for measuring the sulfur ion content of the hydrolysate (15 or 19) after a sufficient amount of antimicrobial has been added. The measuring means may be a sulfur probe or a sulfur sensor arranged or inserted into the process to measure the sulfur content after the antimicrobial compound has been added to the hydrolysate. Figure 1 illustrates suitable positions in the process, when the sulfur ion content is preferably monitored (see illustrative dotted arrows from antimicrobial control means 25). In embodiments, the sulfur ion content is measured inline; i.e. the measurements are performed directly in the process line. The sulfur ion content may be measured inline or online. If the sulfur ion content is measured online, a sample is diverted from the process line, e.g. in a a bypass loop from the main process line (not shown). Means for measuring the sulfur ion content may also (or alternatively) be arranged within the fermentation vessel 23 for securing an efficient and infection free fermentation.

If the fermentation vessel 23 also comprises means to control the microbial fermentation by means for measuring a residual sugar indicator parameter, RSI, the content of sulfur may be measured simultaneously with such parameter.

After a storage stable hydrolysate has been formed, i.e. after the addition of antimicrobial to the hydrolysate stream 15, or optionally hydrolysate stream 19, the hydrolysate may be removed from the process and stored during a period of time, before being used for subsequent fermentation in the fermentation vessel 23.

Although figure 1 illustrates a system 1 for reducing and/or controlling microbial contamination in a separate hydrolysis and fermentation (SHF) process, the same components are used in a system for forming a storage stable hydrolysate (apart from the fermentation vessel 23 and product recovery unit 24).

Terms, definitions and embodiments of the first aspect of the present disclosure apply mutatis mutandis to the other aspects of the present disclosure, and vice versa.

### Example 1: Bacterial contamination in a non-separated lignocellulosic hydrolysate

Non-detoxified spruce hydrolysate (with added 2 g/l yeast extract; 0,5% sodium phosphate; 0,5 g/l diammonium phosphate; 2 g/l peptone and diluted with water to 30 g/l glucose) was filter sterilized and used to represent feed media. An inoculum was prepared using MRS media and the same contaminated sample as used in previous experiments, and was incubated at 37°C overnight (16 hours). Sodium sulfite was added at concentrations of 0 mM, 5 mM, 10 mM, 20 mM and 40 mM, respectively, to 25 ml serum bottles with 22,5 ml of the feed media. After inoculation, the bottles were placed on a multiple magnetic stirring plate at room temperature, with samples taken periodically and analyzed using HPLC.

The hydrolysate used may represent a feed stream where no separation of the pretreatment liquid and solid component has taken place. In other words, hydrolysis and fermentation inhibitors are still present in the feed stream.

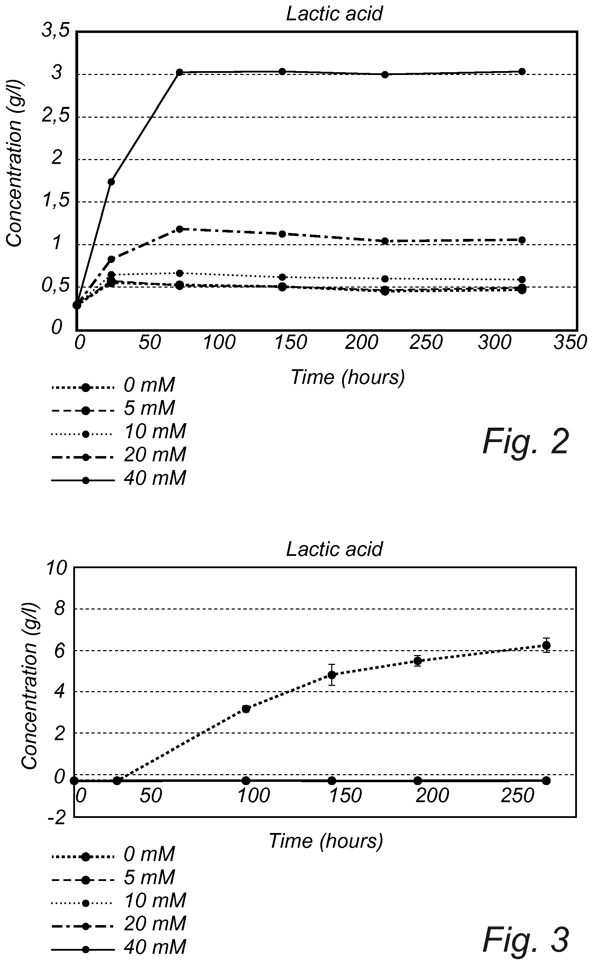

As illustrated in figure 2, the addition of an amount of 5 to 40 mM of sodium sulfite has no antimicrobial effect on the hydrolysate. Instead, the sulfite added acts as a detoxifying agent on the fermentation inhibitors still present in the hydrolysate. The result is an increased bacterial growth when sodium sulfite is added.

### Example 2: Controlling bacterial contamination in a sugar-rich hydrolysate

The aim of this experiment was to test the potential of sodium sulfite to control bacterial contamination in the storage tanks which contain sugar-rich hydrolysate streams before they are used in fermentations. The hydrolysate is representative of a hydrolysate according to the present disclosure.

To test the theory, synthetic media was made containing about 90 g/l glucose, 0,5% sodium phosphate/phosphoric acid buffer (pH 5.5), 0,5 g/l diammonium phosphate, 2 g/l yeast extract and 2 g/l peptone. This solution (45 ml) was added to 50 ml serum bottles, along with a magnetic stirrer bar, and autoclaved, where after sodium sulfite at concentrations of 5, 10, 20 and 40 mM was added, while some bottles had no sulfite added, which served as control (0 mM). In an Erlenmeyer flask, 150 ml MRS media was inoculated using a sample previously collected from a contaminated fermentation at SEKAB, previously showed to involve lactic acid bacteria. This culture was incubated at 37°C for 36 hours and used to inoculate all the serum bottles (10% v/v). The serum bottles were then sealed with an airtight septum and a metal ring clamp and two needles were inserted through the septum to allow for sampling. The serum bottles were placed on a magnetic stirrer plate at room temperature (since storage tanks are typically kept at room temperature), with stirring at 100 rpm. Samples were taken periodically and analyzed for glucose and lactic acid using HPLC. Experiments were performed in triplicate. The experiments were continued for 11 days.

The only growth that took place in the experiment was in the control bottles, both based on the visual evaluation, which indicated no growth or turbidity in any other bottles, as well as the lactic acid data obtained with HPLC analysis. This seems to support the theory that sodium sulfite inhibits the growth of lactic acid bacteria under these conditions. As illustrated in figure 3, sodium sulfite at all concentrations tested (5, 10, 20 and 40 mM) inhibited the growth of lactic acid bacteria (the concentration of lactic acid was 0 g/1).

### Example 3: Controlling bacterial contamination in a sugar-rich hydrolysate comprising 10% non-detoxified spruce hydrolysate

Experiments in this example were performed according to the description in example 2 with the difference that the sugar-rich hydrolysate contained 10 % (v/v) non-detoxified spruce hydrolysate. This was done to simulate sugar-rich hydrolysate with some inhibitory impurities present that may arise from insufficient separation and/or washing.

In the "Experiment A" samples, serum bottles were inoculated with Lactobacillus sp. at time 0 hours and incubated at room temperature for 21 days. Samples were taken periodically and analyzed for glucose and lactic acid (not shown here) using HPLC.

In the "Experiment B" samples, serum bottles were inoculated twice - firstly on the first day and again two weeks later, to simulate a re-infection and test the longevity of the inhibitory effect of sodium sulfite.

In Experiment "C", the third group of serum bottles were inoculated after two weeks of storage at room temperature and inoculated with Lactobacillus after 14 days.

Table 1 illustrates the glucose consumption at different time points for experiments A-C.

**Table 1: Glucose concentration at different time points**

| **Glucose (g/L)** | | | | | |
|---|---|---|---|---|---|
| | **Sample** | **0h** | **168h** | **336h** | **508h** |
| Experiment A | 0 mM | 84,1 | 79,3 | 77,5 | 74,1 |
| | 5 mM | 83,8 | 71,9 | 66,3 | 65,9 |
| | 10 mM | 84,8 | 72,9 | 64,6 | 63,8 |
| | 20 mM | 83,4 | 75,2 | 70,1 | 67,2 |
| | 40 mM | 82,0 | 80,1 | 77,0 | 82,5 |
| Experiment B | 0 mM | 83,3 | 69,1 | 69,0 | 71,2 |
| | 5 mM | 83,8 | 72,6 | 66,8 | 69,0 |
| | 10 mM | 81,7 | 71,5 | 67,9 | 73,4 |
| | 20 mM | 83,1 | 69,0 | 72,4 | 72,1 |
| | 40 mM | 85,2 | 86,6 | 86,2 | 86,3 |
| Experiment C | 0 mM | 82,1 | 81,1 | 83,8 | 78,8 |
| | 5 mM | 85,4 | 84,8 | 83,4 | 77,1 |
| | 10 mM | 84,8 | 81,7 | 81,9 | 75,5 |
| | 20 mM | 85,9 | 82,3 | 83,5 | 79,5 |
| | 40 mM | 80,9 | 81,0 | 85,6 | 82,6 |

Growth was observed in all three experiments for all samples except in those containing 40 mM sodium sulfite, indicated by the glucose consumption in these samples (see table 1). Similar to example 1, a detoxification effect was observed in the samples containing 5-20 mM sodium sulfite, which grew faster than the sample with no sulfite added (0 mM). Thus, in contrast to example 1, where addition of sodium sulfite resulted in a detoxification effect and facilitated growth of Lactobacillus sp., sodium sulfite levels of 40 mM instead introduced an antimicrobial effect in this example. While 10% (v/v) of lignocellulosic hydrolysate containing impurities may be considered a moderate to low level of impurities, this examples clearly shows the importance of creating sugar-rich hydrolysates with a minimum of inhibitory compounds. Presence of such compounds results in a reaction between the sodium sulfite and the inhibitory compounds, causing a detoxification of the hydrolysate and facilitating bacterial growth rather than introducing an antimicrobial effect. To displace the effect of detoxification caused by sodium sulfite addition (even at moderate levels of impurities) requires higher amounts of sodium sulfite, i.e. 40 mM in this experiment compared to 5 mM in examples 2 and 4.

### Example 4: Storage of a sugar-rich hydrolysate

Storage of sugar-rich hydrolysate with addition of 5-40 mM was performed in accordance with the description of example 2. In this example, the samples were prepared and handled as in example 2. In other words, the "Experiment A" samples in this example are identical to those of example 2, and these were used as a reference for the "Experiment B" and "Experiment C" samples, as well as for validating the results of example 2.

The "Experiment B" samples were inoculated with a Lactobacillus sp. at time 0 hours, and again after 336 hours. This was done to test the infection resistance of samples that were exposed to more than one culture of Lactobacillus sp; i.e. samples that were re-infected after two weeks.

The samples in "Experiment C" were stored at ambient room temperature for 2 weeks, after which Lactobacillus was added to the flasks, after 336 hours. Samples were incubated for another 7 days and a total of 21 days as for the other two experiments. Table 2 illustrates the glucose levels at various time points throughout the storage period.

**Table 2: Glucose concentration at different time points**

| **Glucose (g/L)** | | | | | |
|---|---|---|---|---|---|
| | **Sample** | **0h** | **172h** | **340h** | **510h** |
| Experiment A | 0 mM | 84,4 | 75,1 | 63,9 | 38,3 |
| | 5 mM | 83,3 | 84,0 | 83,5 | 83,2 |
| | 10 mM | 84,1 | 85,3 | 82,8 | 82,2 |
| | 20 mM | 83,7 | 85,6 | 86,8 | 84,7 |
| | 40 mM | 81,5 | 83,2 | 83,2 | 83,6 |
| Experiment B | 0 mM | 83,4 | 73,9 | 62,6 | 37,2 |
| | 5 mM | 82,3 | 82,9 | 82,6 | 78,3 |
| | 10 mM | 82,9 | 84,3 | 81,5 | 80,8 |
| | 20 mM | 82,8 | 74,7 | 86,0 | 83,3 |
| | 40 mM | 80,2 | 82,1 | 82,0 | 82,5 |
| Experiment C | 0 mM | 81,7 | 82,4 | 84,6 | 68,7 |
| | 5 mM | 81,2 | 81,6 | 85,5 | 82,0 |
| | 10 mM | 82,2 | 81,8 | 84,0 | 82,4 |
| | 20 mM | 80,8 | 80,3 | 84,5 | 83,3 |
| | 40 mM | 82,0 | 81,6 | 86,2 | 82,0 |

The results of "Experiment A" verified the effect observed in example 2; i.e. that bacterial growth occurs in the absence of sodium sulfite. Flasks with added sodium sulfite showed no glucose consumption during three weeks, indicating that no bacteria were able to grow, and thus no consumption of sugar was evident.

The results of "Experiment B" are the same as "Experiment A", but allows for a second effect of sodium sulfite addition to be demonstrated; i.e. that a first addition of Lactobacillus sp. does not decrease the effect of sodium sulfite significantly at any concentration above 5 mM to which a second addition of Lactobacillus sp. was made. No signs of bacterial growth was observed during 21 days for experiments containing 5 mM or more of sodium sulfite.

The results from "Experiment C" demonstrate that 14 days of storage of a sugar-rich hydrolysate at room temperature containing sodium sulfite concentrations above 5 mM does not decrease the antimicrobial effect of sodium sulfite. As seen in table 2, no growth was observed in such samples compared to the reference with no sodium sulfite addition. Hence, sodium sulfite at all concentrations tested (5, 10, 20 and 40 mM) inhibited the growth of Lactobacillus sp. over three weeks of incubation while samples containing no sulfite allowed for bacterial growth (see table 2). These results indicate that storage for at least three weeks can be achieved with additions of 5-40 mM sodium sulfite when no compounds that can react with the sodium sulfite are present (compared to e.g. example 3). Presence of such compounds rather results in detoxification effects demonstrated in example 1 and in example 3.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A method for forming a storage stable hydrolysate from a lignocellulosic material comprising:
a) pretreating said lignocellulosic material to form a pretreated lignocellulosic composition comprising a solid component and a liquid component; said solid component comprising at least lignin and cellulose,
b) removing at least 80%, preferably at least 90% of said liquid component from said pretreated lignocellulosic composition to form a separated lignocellulosic component
c) diluting said lignocellulosic component with a dilution liquid to form an aqueous slurry,
d) subjecting said aqueous slurry to hydrolysis in the presence of at least one saccharification enzyme to form a hydrolysate,
e) adding an antimicrobial compound to said hydrolysate in an amount sufficient to form a storage stable hydrolysate, wherein said antimicrobial compound comprises at least one sulfur oxyanion.

2. The method of claim 1, wherein said aqueous slurry formed in said step c) has a suspended solids content of from 10 % to 35 % by weight.

3. The method of claim 1 or claim 2, wherein said hydrolysis step d) is performed in the presence of oxygen.

4. The method of any one of the preceding claims, wherein the concentration of said antimicrobial compound in said hydrolysate is between 1 and 100 mM, preferably between 5 and 25 mM.

5. The method of any one of the preceding claims, wherein said antimicrobial compound is selected from dithionite and sulfite.

6. The method of any one of the preceding claims, further comprising the step of
- subjecting said hydrolysate formed in said step d) or said step e) to separation to remove at least a portion of residual solid components formed during said hydrolysis.

7. The method of any one of the preceding claims, further comprising the step of controlling the amount of antimicrobial compound in said storage stable hydrolysate by
- measuring the sulfur ion content in said storage stable hydrolysate,
- comparing said sulfur ion content with a reference sulfur value, and optionally,
- adding an additional amount of said antimicrobial compound if said sulfur ion content is lower than said reference sulfur value.

8. A hydrolysate formed by the method of any one of claims 1-7.

9. Use of the hydrolysate of claim 8 to reduce and/or control microbial contamination during storage and/or fermentation of a target chemical.

10. A method for reducing and/or controlling microbial contamination in a separate hydrolysis and fermentation (SHF) process comprising:
- providing a storage stable hydrolysate according to claim 8 or formed by the method of any one of claims 1-7,
- subjecting said storage stable hydrolysate to fermentation.

11. The method of claim 10, wherein said fermentation is a fed-batch or a continuous fermentation.

12. The method of claim 10 or claim 11, wherein said method further comprises the step of storing said hydrolysate at least three weeks, preferably at least five weeks, more preferably at least eight weeks at room temperature prior to fermentation.

13. A system for forming a storage stable hydrolysate, wherein said system comprises:
- a pretreatment unit (2) for pretreating said lignocellulosic material to form a pretreated lignocellulosic composition comprising a liquid component and a solid component, said solid component comprising at least lignin and cellulose,
- means (7) for removing at least 80%, preferably at least 90% of said liquid component from said pretreated lignocellulosic composition to form a separated lignocellulosic component,
- means (10) for diluting said lignocellulosic component with a dilution liquid to form an aqueous slurry,
- a hydrolysis unit (12) arranged to receive said aqueous slurry and to hydrolyze said aqueous slurry in the presence of at least one saccharification enzyme to form a hydrolysate,
- means (18) for adding an antimicrobial compound to said hydrolysate to form a storage stable hydrolysate, wherein said antimicrobial compound comprises at least one sulfur oxyanion.

14. A system according to claim 13, wherein said hydrolysis unit (12) is configured to receive an air stream through an inlet (21) of said hydrolysis unit (12) and to discharge said air stream by means of an outlet (22) of said hydrolysis unit (12).

15. A system according to claim 13 or claim 14, further comprising means (25) for controlling the amount of antimicrobial compound in said storage stable hydrolysate, wherein said means (25) comprises
- means for measuring the sulfur ion content of said storage stable hydrolysate, and optionally
- means for adding additional antimicrobial compound in case the sulfur ion content is lower than a reference value.

16. A system (1) for reducing and/or controlling microbial contamination in a separate hydrolysis and fermentation (SHF) process comprising:
- a system for forming a storage stable hydrolysate according to any one of claims 13-15
- at least one fermentation vessel (23) arranged downstream of and in fluid connection with said system for forming a storage stable hydrolysate.

17. Use of dithionite or sulfite for decreasing microbial contamination of a hydrolysate formed from a lignocellulosic material.
